Europäisches Patentamt

European Patent Office

Office européen des brevets

(19)

(11) Veröffentlichungsnummer : **0 070 432**
**B1**

(12) # EUROPÄISCHE PATENTSCHRIFT

(45) Veröffentlichungstag der Patentschrift :
03.04.85

(51) Int. Cl.⁴ : **C 07 C102/00, C 07 C103/90**

(21) Anmeldenummer : **82105867.4**

(22) Anmeldetag : **01.07.82**

(54) **Verfahren zur Herstellung von gereinigtem Tetraacetylethylendiamin (TAED).**

(30) Priorität : **11.07.81 DE 3127435**

(43) Veröffentlichungstag der Anmeldung :
**26.01.83 Patentblatt 83/04**

(45) Bekanntmachung des Hinweises auf die Patenterteilung : **03.04.85 Patentblatt 85/14**

(84) Benannte Vertragsstaaten :
**DE GB**

(56) Entgegenhaltungen :
**DE-A- 2 052 822**
**DE-A- 3 024 694**

(73) Patentinhaber : **BAYER AG**
**Konzernverwaltung RP Patentabteilung**
**D-5090 Leverkusen 1 Bayerwerk (DE)**

(72) Erfinder : **Blank, Heinz Ulrich, Dr.**
**Am Geusfelde 35**
**D-5068 Odenthal (DE)**
Erfinder : **Langenfeld, Norbert, Dr.**
**Gerstenkamp 2**
**D-5000 Koeln 80 (DE)**
Erfinder : **Fischer, Rolf, Dr.**
**An Gr. St. Martin 8**
**D-5000 Koeln 1 (DE)**
Erfinder : **Schnegg, Peter, Dr.**
**Heidberger Strasse 44**
**D-5068 Odenthal (DE)**

EP 0 070 432 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von reinem TAED aus TAED enthaltenden Gemischen durch Dispersion solcher Gemische in einem Suspensionsmittel.

Rohes TAED wird nach bereits bekannten Verfahren durch Acetylierung von Ethylendiamin oder von acetyliertem Ethylendiamin, beispielsweise N,N'-Diacetylethylendiamin (DAED) mit Acetanhydrid hergestellt (DE-OS 28 32 021 ; DE-OS 28 16 174). Ein anderes Verfahren (DE-AS 21 33 458) setzt Ethylendiamin mit Essigsäure zunächst zum DAED und dann mit Acetanhydrid oder Keten zum TAED um. Die Aufarbeitung und Reinigung der Produktgemische geschieht in der Regel dadurch, daß TAED durch Abkühlen zum Auskristallisieren gebracht und durch Filtrieren oder Zentrifugieren von der Mutterlauge aus Acetanhydrid und Essigsäure abgetrennt wird. Nachteilig bei diesem Verfahren ist, daß das so erhaltene TAED oft noch verfärbt ist und daß ein Teil des TAED sowie DAED und N,N,N'-Triacetylethylendiamin (TriAED) neben unerwünschten Verunreinigungen in der Mutterlauge verbleiben. Die Verunreinigungen müssen vor einer Rückführung der Mutterlauge in die Acetylierungsreaktion zumindest teilweise entfernt werden. So wird nach DE-AS 21 18 281 nur ein Teil der Mutterlauge zurückgeführt und ein anderer Teil wird ausgeschleust, um eine Anreicherung der Verunreinigungen zu vermeiden. Nach DE-OS 28 16 174 werden die in der Mutterlauge enthaltenen Verunreinigungen durch Adsorption an Bleicherde oder Aktivkohle oder durch eine Destillation des Mutterlaugenrückstandes entfernt.

Eine weitere Möglichkeit zur Reinigung des Acetylierungsgemisches wird in DE-OS 28 32 021 beschrieben : Hier wird aus dem Produktgemisch zunächst Acetanhydrid destillativ entfernt. Dann wird das TAED aus dem Acetylierungsgemisch bei einem Druck von 1 bis 10 mbar destilliert. Das so erhaltene TAED ist zwar von befriedigender Farbqualität, es kann aber weiterhin nicht-umgesetztes DAED und vor allem TriAED enthalten, die unter den Destillationsbedingungen ebenfalls flüchtig sind. Bereits durch geringe Mengen dieser Verunreinigungen wird der Schmelzpunkt des TAED deutlich herabgesetzt, und die Lagerstabilität verschlechtert sich.

Es wurde nun ein Verfahren zur Herstellung von gereinigtem Tetraacetylethylendiamin (TAED) gefunden, das dadurch gekennzeichnet ist, daß man TAED enthaltende Produktgemische, die bei der Herstellung von TAED durch Acetylierung von Ethylendiamin oder acetyliertem Ethylendiamin erhalten wurden, in den Suspensionsmitteln Wasser, wäßrigen Lösungen von neutralen Salzen, Carbonsäuren oder Carbonsäureanhydriden, die einzeln oder als Gemisch mehrerer angewandt werden und die eine Temperatur von − 20 bis + 100 °C haben, dispergiert, wobei die Teilchen des Produktgemisches vor oder während des Dispergierens auf eine durchschnittliche Größe von 1-2 000 µm gebracht werden, und nach einer Verweilzeit von 0,1 Minuten bis zu mehreren Tagen das TAED nach bekannten Methoden vom Suspensionsmittel isoliert.

Als Reinigungseffekt im erfindungsgemäßen Verfahren wird eine Absenkung der menge an Gesamtverunreinigungen auf einen Wert von 0 bis zu etwa 50 % der vorher vorhandenen Gesamtverunreinigungen erzielt.

Für die einzelnen Verunreinigungen können dabei durchaus abweichende Werte beobachtet werden. So wird beispielsweise beobachtet, daß bei Verunreinigungen, die nur in einer geringen absoluten Mengen vorhanden sind, die Absenkung in manchen Fällen nur auf 40 bis 80 % des vorhandenen Wertes erzielt wird.

Dieser geringere Effekt bei den mengenmäßig kleinen Verunreinigungen wird jedoch durch den Effekt bei den mengenmäßig großen Verunreinigungen überkompensiert. Hier lassen sich Absenkungen auf Werte von 0 bis etwa 40 %, bevorzugt von 0 bis 30 % erzielen.

Die Suspensionsmittel sind die in den Ansprüchen genannten anorganischen und organischen Flüssigkeiten. Die organischen Flüssigkeiten sind Carbonsäuren, wie z. B. Ameisensäure, Essigsäure, Propionsäure und Buttersäure, oder ihre Anhydride, wie z. B. Acetanhydrid.

Die Suspensionsmittel können auch Mischungen solcher Stoffe sein, die miteinander nicht reagieren oder die zu Stoffen reagieren, die in der genannten Aufzählung enthalten sind. Beispielsweise reagiert Wasser mit Acetanhydrid zu der ebenfalls in der Aufstellung genannten Essigsäure.

Bevorzugte Suspensionsmittel sind Wasser, Essigsäure, Essigsäureanhydrid, Wasser/Essigsäure- bzw. Essigsäure/Essigsäureanhydrid-Gemische. In besonders bevorzugter Weise wird Wasser als Suspensionsmittel eingesetzt.

Die Temperatur, mit der das Suspensionsmittel eingesetzt wird, hängt von den allgemeinen Eigenschaften des Suspensionsmittels, wie Schmelz- und Siedepunkt, ab. Allgemein sei eine Temperatur von − 20 °C bis + 100 °C genannt. Bevorzugt wird eine Temperatur von 0 bis 60 °C, besonders bevorzugt von 10 bis 50 °C eingestellt. Bei den bevorzugten Varianten der Temperatureinstellung ist es vielfach vorteilhaft, daß die Suspension nicht zusätzlich gekühlt oder geheizt werden muß. In ganz besonders bevorzugter Weise sei als Temperatur für das Suspensionsmittel Wasser eine solche von 0 bis 50 °C, für Essigsäure eine solche von 17 bis 50 °C genannt.

Das Gewichtsverhältnis zwischen dem Suspensionsmittel und dem TAED enthaltenden Gemisch liegt im allgemeinen zwischen 0,5 bis 100 zu 1, bevorzugt von 1 bis 50 zu 1, besonders bevorzugt von 2 bis 10 zu 1.

Das TAED enthaltende Gemisch wird mit einer durchschnittlichen Teilchengröße von 1 bis 2 000 µm,

bevorzugt von 10 bis 500 μm, besonders bevorzugt von 50 bis 250 μm, im Suspensionsmittel dispergiert. Hierbei ist es möglich, die Teilchen in die gewünschte durchschnittliche Größe zu bringen, bevor sie suspendiert werden oder auch während der Suspendierung. Ein das TAED enthaltendes Gemisch im gewünschten Korngrößenspektrum kann beispielsweise direkt in das Suspensionsmittel gegeben werden. Eine weitere Ausführungsform besteht darin, das feste, TAED enthaltende Gemisch im Suspensionsmittel zu mahlen (Naßmahlung) und so die gewünschte Korngrößenverteilung zu erzielen. Geräte, mit denen Feststoffe naß gemahlen werden können, sind dem Fachmann bekannt, beispielsweise Homogenisatoren, Turborührer, Kugelmühlen und weitere (Ullmann's Encyclopädie der technischen Chemie, 4. Auflage 1972, Band 2, Seiten 2 bis 23).

Eine weitere Möglichkeit besteht darin, eine Schmelze des das TAED enthaltenden Gemisches in das Suspensionsmittel so einzutragen, daß feste Partikel in der gewünschten Korngrößenverteilung in der Suspension erhalten werden. Beispielsweise kann die Suspension durch Verdüsen der Schmelze des das TAED enthaltenden Gemisches in das Suspensionsmittel hergestellt werden. Ebenso kann die Dispergierung der Schmelze mit Hilfe eines Rotor-Stator-Systems im Suspensionsmittel hergestellt werden.

Die Verweilzeit des dispergierten, TAED enthaltenden Gemisches im Suspensionsmittel nach der Dispergierung kann 0,1 Minute bis zu mehreren Tagen betragen. Eine bevorzugte Verweilzeit beträgt von 0,5 Minuten bis 24 Stunden, besonders bevorzugt von 2 Minuten bis 12 Stunden, ganz besonders bevorzugt von 5 Minuten bis 6 Stunden.

Als TAED enthaltende Gemische kommen Produktgemische in Frage, die neben TAED, Essigsäureanhydrid und/oder wenig Essigsäure noch unterschiedliche Mengen an TriAED, DAED und anderen Verunreinigungen enthalten können. Die Herstellung dieser Produktgemische kann beispielsweise so erfolgen wie in den bereits genannten Patentanmeldungen (DE-AS 21 18 281, DE-AS 21 33 458 oder DE-OS 28 16 174). Vorteilhaft werden vor Anwendung des erfindungsgemäßen Verfahrens überschüssiges Essigsäureanhydrid und/oder Essigsäure durch Destillation bis auf geringe Mengen entfernt. Bevorzugt wird der Rückstand einer solchen destillativen Lösungsmittelentfernung im Vakuum destilliert. Der TAED-Gehalt eines so erhaltenen Gemisches für die erfindungsgemäße Suspendierung kann in weiten Grenzen schwanken, beispielsweise zwischen 30 und 99,9 Gew.-%, vorzugsweise von 50 bis etwa 99 Gew.-%, insbesondere 85-98 Gew.-%. Diese Gemische enthalten etwa 0,1-50 Gew.-% an TriAED und etwa 0,01-20 Gew.-% an DAED, vorzugsweise etwa 1-40 Gew.-% an TriAED und 0,1-10 % an DAED, insbesondere etwa 1,8-12 % an TriAED und 0,2-3 Gew.-% an DAED. Bei hohen Gehalten an TAED, beispielsweise über 95 Gew.-%, und unter 4 % an TriAED sowie unter 1 % an DAED kann es günstig sein, Wasser als Suspensionsmittel zu verwenden und anschließend das Suspensionsmittel ohne hohe Produktverluste zu verwerfen. Bei niedrigen Gehalten an TAED, beispielsweise unter 95 Gew.-%, haben beispielsweise Acetanhydrid oder Gemische hieraus den Vorteil, daß das im Suspensionsmittel verbliebene DAED, TriAED und auch etwas TAED ohne weitere Aufarbeitung in die Acetylierung zurückgeführt werden kann.

Die Isolierung des hergestellten reinen TAED aus der Suspension kann nach bekannten Methoden, beispielsweise durch Filtration oder Zentrifugieren, erfolgen.

Es kann vorteilhaft sein, das erfindungsgemäße Verfahren mit weiterer Destillation oder Sublimation zu kombinieren. Beispielsweise kann, wie bereits beschrieben, das nach der Acetylierung von überschüssigem Acetylierungsmittel befreite, TAED enthaltende Gemisch vor der erfindungsgemäßen Suspendierung destilliert oder sublimiert werden, wobei DAED, TriAED und TAED als farbloses Destillat oder Sublimat übergehen, während die verfärbenden Verunreinigungen im Rückstand verbleiben. Die Abtrennung von verfärbenden Verunreinigungen oder höhersiedenden Nebenprodukten erfolgt vorzugsweise durch Destillation. Selbstverständlich ist es auch möglich, die Destillation oder Sublimation im Anschluß an die erfindungsgemäße Suspendierung durchzuführen. Je nach Qualitätsanforderung kann auf eine zusätzliche Destillation oder Sublimation jedoch auch ganz verzichtet werden, zumal je nach Suspensionsmittel auch durch die alleinige Suspendierung die verfärbenden Bestandteile entfernt werden können.

Es muß als ausgesprochen überraschend bezeichnet werden, daß eine Reinigung des TAED durch Suspendierung möglich ist, da im allgemeinen vergleichbare Reinigungseffekte nur durch Umkristallisieren erreicht werden können. So ist es nach der bisherigen Patentliteratur zur Erzielung eines weitgehend von DAED und TriAED freien TAED erforderlich, entweder das TAED aus der Acetylierungslösung auszukristallisieren oder den Umsatz zu TAED praktisch quantitativ zu gestalten. Weiterhin ist es überraschend, daß das TAED beim erfindungsgemäßen Eintrag oder Verbleiben in Wasser oder anderen Suspensionsmitteln, mit denen es reagieren kann, chemisch unverändert bleibt, selbst wenn es über mehrere Stunden in der Suspension verbleibt. Dies ist überraschend, da nach US 4 087 369 TAED bereits beim Stehen an feuchter Luft zur Zersetzung neigt.

Das erfindungsgemäße Verfahren ermöglicht die Herstellung von reinem TAED ohne eine Kristallisation aus dem Reaktionsansatz und ohne daß der Umsatz vollständig sein muß. Weitere Vorteile des Verfahrens sind :

1. Durch die Art der Suspendierung kann auf einfache Weise ein gewünschtes Teilchengrößenspektrum erhalten werden, so daß ein Zerkleinerungs- und ein Reinigungsschritt in einem Arbeitsgang möglich sind.

2. Bei der Verwendung von Wasser als Suspensionsmittel hat das Verfahren gegenüber der Isolierung durch Auskristallisieren den Vorteil, daß bereits durch die Suspendierung das TAED von evtl.

anhaftendem Acetanhydrid befreit werden kann.

3. Eine Rückführung von DAED und TriAED in die Acetylierungsstufe ist ohne großen Aufwand möglich, bei Verwendung von Acetanhydrid oder Essigsäure als Suspensionsmittel sogar gelöst in diesem Suspensionsmittel.

4. Durch die Art der Suspendierung besteht die Möglichkeit, die Form und Größe der TAED-Partikel zu beeinflussen, was hinsichtlich Anwendung, Lagerbeständigkeit und Wirksamkeit des Produktes von Bedeutung ist (vgl. US-PS 4 087 369).

5. Die Herstellung von reinem TAED nach dem erfindungsgemäßen Verfahren erfordert sehr geringen apparativen Aufwand ; dies gilt besonders dann, wenn bei einer Kombination TAED-Destillation/Suspendierung das flüssige Destillat anschließend in das Suspensionsmittel eingebracht wird.

6. Gegenüber einer Reinigung durch Umkristallisation bietet das Verfahren den Vorteil niedrigerer Energiekosten.

TAED wird als Bleichmittelaktivator für Waschmittel verwendet. Es setzt in diesen Waschmitteln die für die Bleichmittelwirkung notwendige Temperatur herab.

## Beispiel 1

50 g Roh-Tetraacetyl-ethylendiamin (TAED), die durch Destillation aus einem nicht vollständig umgesetzten Acetylierungsansatz gewonnen wurden, werden in 200 g Wasser von 20 °C in einer Kugelmühle 5 Stunden gemahlen. Temperatur nach der Naßmahlung 25 °C. Die Suspension wird abgesaugt und bei 40 °C im Vakuumtrockenschrank bis zur Gewichtskonstanz getrocknet. Das Ergebnis zeigt die Tabelle I.

## Beispiel 2

50 g Roh-TAED, wie in Beispiel 1, werden in 200 g Acetanhydrid von 20 °C in einer Kugelmühle 5 Stunden gemahlen. Temperatur nach der Naßmahlung 27 °C. Die Suspension wird wie in Beispiel 1 aufgearbeitet. Das Ergebnis zeigt die Tabelle I.

## Beispiel 3

50 g Roh-TAED werden in 200 g Essigsäure von 20 °C in einer Kugelmühle 5 Stunden gemahlen. Temperatur nach der Naßmahlung 25 °C. Die Suspension wird wie in Beispiel 1 aufgearbeitet. Das Ergebnis zeigt die Tabelle I.

(Siehe Tabelle I Seite 5 f.)

4

Tabelle I

| Bei-spiel | | Gewicht g | Schmp. °C | TAED % | Gehalt TriAED* % | DAED* % | Anteil des eingesetzten TAED % |
|---|---|---|---|---|---|---|---|
| 1 | Einsatz | 50 | 142–148 | 89 | 10 | 1 | 100 |
| | Produkt | 45,1 | 151–152 | 97 | 2 | 0,5 | 98,5 |
| | Trockenmasse der Mutterlauge | 4,5 | | 5 | 85 | 10 | 0,5 |
| 2 | Einsatz | 50 | 142–148 | 89 | 10 | 1 | 100 |
| | Produkt | 43,1 | 151–152 | 97 | 2 | 0,5 | 94 |
| | Trockenmasse der Mutterlauge | 6,5 | | 35 | 60 | 4 | 5 |
| 3 | Einsatz | 50 | 137–147 | 87 | 12 | 1 | 100 |
| | Produkt | 38 | 151,5 | 97 | 2 | 0,7 | 84 |
| | Trockenmasse der Mutterlauge | 11,5 | | 61 | 34 | 4 | 16 |

\* TriAED = Triacetyl-ethylendiamin

  DAED  = Diacetyl-ethylendiamin

0 070 432

### Beispiel 4

50 g Roh-TAED werden in 100 g Acetanhydrid von 5 °C in einer Kugelmühle 5 Stunden gemahlen. Temperatur nach der Naßmahlung 12 °C. Die Suspension wird wie in Beispiel 1 aufgearbeitet. Das Ergebnis zeigt die Tabelle II.

### Beispiel 5

50 g Roh-TAED werden in 100 g Wasser von 20 °C in einer Kugelmühle 5 Stunden gemahlen. Temperatur nach der Naßmahlung 27 °C. Die Suspension wird wie in Beispiel 1 aufgearbeitet. Das Ergebnis zeigt die Tabelle II.

### Beispiel 6

In ein Becherglas mit 500 g Wasser von 20 °C taucht ein « Ultra-Turrax® » Turborührer ein. Bei eingeschaltetem Rührer werden 71,5 g einer Roh-TAED-Schmelze von 160 °C innerhalb 30 Sekunden in die Nähe des Rührers gegossen. Die entstandene Suspension hat eine Temperatur von 40 °C und wird wie in Beispiel 1 aufgearbeitet. Das Ergebnis zeigt die Tabelle II.

### Beispiel 7

Das Roh-TAED in den Beispielen 1 bis 5 wird in Anlehnung an DE-AS 2 133 458 wie folgt erhalten :
144 g DAED und 510 g Acetanhydrid werden zum Sieden erhitzt. Innerhalb von 5 Stunden werden über eine 50 cm Füllkörperkolonne 75 g Essigsäure abdestilliert. Aus dem Rückstand werden Essigsäure und Acetanhydrid in Vakuum abdestilliert. Das Roh-TAED erhält man durch Destillation des verbleibenden Rückstandes bei 3 mbar.
Das Roh-TAED in Beispiel 6 wird wie oben beschrieben erhalten mit dem Unterschied, daß anstelle der 75 g Essigsäure 200 g eines Gemisches aus Essigsäure und Acetanhydrid abdestilliert werden.

(Siehe Tabelle II Seite 7 f.)

Tabelle II

| Bei-spiel | | Gewicht g | Schmp. °C | Gehalt | | | Anteil des eingesetzten TAED % |
| | | | | TAED % | TriAED % | DAED % | |
| --- | --- | --- | --- | --- | --- | --- | --- |
| 4 | Einsatz | 50 | 141–148 | 87 | 12 | 1 | 100 |
| | Produkt | 43,0 | 150–151 | 98 | 2 | 0 | 97 |
| | Trockenmasse der Mutter-lauge | 6,8 | | 18 | 76 | 6 | 3 |
| 5 | Einsatz | 50 | 141–148 | 87 | 12 | 1 | 100 |
| | Produkt | 44,2 | 150–151 | 97 | 3 | 0 | 99 |
| | Trockenmasse der Mutter-lauge | 5,4 | | 2 | 83 | 15 | 0,3 |
| 6 | Einsatz | 71,5 | 149–150 | 96 | 3,5 | 0,5 | 100 |
| | Produkt | 67,8 | 151 | 99,5 | 0,5 | 0 | 98,3 |
| | Trockenmasse der Mutter-lauge | 3,0 | | 23 | 65 | 12 | 1,0 |

0 070 432

## Beispiel 8

908 g Roh-TAED, das wie in Beispiel 6 erhalten wurde, wird in einem Stahl-Email-Autoklaven der im Bodenauslaß eine Düse mit 0,5 mm Durchmesser besitzt, unter Stickstoff aufgeschmolzen und innerhalb von 120 sec. in 4 l Wasser von 20 °C eingedüst und mit einem Kreuzbalkenrührer verrührt. Die entstandene Suspension hat eine Temperatur von 41 °C und wird wie in Beispiel 1 aufgearbeitet. Das Ergebnis zeigt Tabelle III.

## Beispiel 9

2 kg Roh-TAED, das wie in Beispiel 6 erhalten wurde, wird in einem Stahl-Email-Autoklaven unter Stickstoff aufgeschmolzen und durch eine Glattstrahldüse mit 0,5 mm Durchmesser und mehreren Bohrungen in einen Prillturm unter $N_2$-Atmosphäre verdüst. Ein Teil des erhaltenen Granulats wird anschließend in der dreifachen Gewichtsmenge Wasser bei 20 °C mit einem Flügelrührer bei 120 Upm 2 Stunden verrührt. Danach wird die Mutterlauge abgesaugt ; das Granulat wird im Vakuumtrockenschrank bei 50 °C und 300 Torr getrocknet. Das Ergebnis zeigt Tabelle III.

Tabelle III

| Bei-spiel | | Gewicht g | Schmp. °C | Gehalt TAED % | TriAED % | DAED % | Anteil des eingesetzten TAED % |
|---|---|---|---|---|---|---|---|
| 8 | Einsatz | 908 | 151 | 97,7 | 1,3 | 1 | 100 |
| | Produkt | 877,5 | 151–152 | 100 | 0 | 0 | 98,9 |
| | Trockenmasse der Mutterlauge | 30,5 | | 25,0 | 51,1 | 18,2 | 0,9 |
| 9 | Einsatz | 136 | 149–151 | 96,4 | 1,6 | 1,7 | 100 |
| | Produkt | 130,2 | 151,5–152,5 | 100 | 0 | 0 | 99,3 |
| | Trockenmasse der Mutterlauge | 5,0 | | 24,4 | 42,8 | 12,8 | 0,7 |

0 070 432

## Ansprüche

1. Verfahren zur Herstellung von gereinigtem Tetraacetylethylendiamin (TAED), dadurch gekennzeichnet, daß man TAED enthaltende Produktgemische, die bei der Herstellung von TAED durch Acetylierung von Ethylendiamin oder acetyliertem Ethylendiamin erhalten wurden, in den Suspensionsmitteln Wasser, wäßrigen Lösungen von neutralen Salzen, Carbonsäuren oder Carbonsäureanhydriden, die einzeln oder als Gemisch mehrerer angewandt werden und die eine Temperatur von − 20 bis + 100 °C haben, dispergiert, wobei die Teilchen des Produktgemisches vor oder während des Dispergierens auf eine durchschnittliche Größe von 1-2 000 μm gebracht werden, und nach einer Verweilzeit von 0,1 Minuten bis zu mehreren Tagen das TAED nach bekannten Methoden vom Suspensionsmittel isoliert.

2. Verfahren nach Anspruch 1, dadurch gekennzeichnet, daß man als Suspensionsmittel Wasser, Essigsäure, Essigsäureanhydrid, eine Wasser/Essigsäure- oder Essigsäure/Essigsäureanhydrid-Mischung verwendet.

3. Verfahren nach Anspruch 1 und 2, dadurch gekennzeichnet, daß man als Suspensionsmittel Wasser verwendet.

4. Verfahren nach Anspruch 1 bis 3, dadurch gekennzeichnet, daß das Gewichtsverhältnis zwischen Suspensionsmittel und dem TAED enthaltenden Produktgemisch von 0,5 bis 100 zu 1 liegt.

5. Verfahren nach Anspruch 1 bis 4, dadurch gekennzeichnet, daß das TAED enthaltende Produktgemisch in fester Form in das Suspensionsmittel eingebracht wird.

6. Verfahren nach Anspruch 1 bis 4, dadurch gekennzeichnet, daß eine Schmelze, ein Destillat oder ein Sublimat des das TAED enthaltenden Produktgemisches in das Suspensionsmittel eingebracht wird.

7. Verfahren nach Anspruch 1 bis 6, dadurch gekennzeichnet, daß das TAED enthaltende Produktgemisch im Suspensionsmittel auf eine durchschnittliche Teilchengröße im Bereich von 1 bis 2 000 μm gebracht wird.

8. Verfahren nach Anspruch 1 bis 7, dadurch gekennzeichnet, daß die im Suspensionsmittel zurückbleibenden Begleitstoffe des TAED wieder in die Acetylierung zur Herstellung des TAED zurückgeführt werden.

9. Verfahren zur Herstellung von gereinigtem TAED, dadurch gekennzeichnet, daß man

1. TAED enthaltende Produktgemische, die bei der Herstellung von TAED durch Acetylierung von Ethylendiamin oder acetyliertem Ethylendiamin erhalten würden, durch Sublimation oder vorzugsweise Destillation von verfärbenden und/oder hochsiedenden Verunreinigungen befreit und

2. die als Sublimat oder Destillat erhaltenen Gemische, enthaltend 30-99,9 Gew.-% TAED, etwa 0,1-50 Gew.-% TriAED und etwa 0,01-20 Gew.-% DAED, in den Suspensionsmitteln Wasser, wäßrigen Lösungen von neutralen Salzen, Carbonsäuren oder Carbonsäureanhydriden, die einzeln oder als Gemisch mehrerer angewandt werden, bei − 20 bis + 100 °C dispergiert, wobei die Teilchen des Produktgemisches vor oder während des Dispergierens auf eine durchschnittliche Größe von 1-2 000 μm gebracht werden, und nach einer Verweilzeit von 0,1 Minuten bis zu mehreren Tagen das TAED nach bekannten Methoden vom Suspensionsmittel isoliert.

## Claims

1. Process for the preparation of purified tetraacetylethylenediamine (TAED), characterised in that product mixtures containing TAED which have been obtained in the preparation of TAED by the acetylation of ethylenediamine or acetylated ethylenediamine, are dispersed in the suspending agents water, aqueous solutions of neutral salts, carboxylic acids or carboxylic acid anhydrides, which are used by themselves or as mixtures of more than one of these and which have a temperature of − 20 to + 100 °C, the particles of the product mixture being brought to an average size of 1-2,000 μm before or during the dispersion, and after a residence time of from 0.1 minutes to several days the TAED is isolated from the suspending agent by known methods.

2. Process according to Claim 1, characterised in that water, acetic acid, acetic anhydride, a water/acetic acid mixture or an acetic acid/acetic anhydride mixture is used as the suspending agent.

3. Process according to Claim 1 and 2, characterised in that water is used as the suspending agent.

4. Process according to Claim 1 to 3, characterised in that the weight ratio between the suspending agent and the product mixture containing TAED is 0.5 to 100 : 1.

5. Process according to Claim 1 to 4, characterised in that the product mixture containing TAED is introduced in a solid form into the suspending agent.

6. Process according to Claim 1 to 4, characterised in that a melt, a distillate or a sublimate of the product mixture containing the TAED is introduced into the suspending agent.

7. Process according to Claim 1 to 6, characterised in that the product mixture containing the TAED is brought, in the suspending agent, to an average particle size in the range of 1 to 2,000 μm.

8. Process according to Claim 1 to 7, characterised in that the substances accompanying the TAED which remain in the suspending agent are passed back to the acetylation for the preparation of the TAED.

9. Process for the preparation of purified TAED, characterised in that

1. product mixtures containing TAED which have been obtained in the preparation of TAED by the acetylation of ethylenediamine or acetylated ethylenediamine are freed from discolouring and/or high-boiling impurities by sublimation or preferably distillation and

2. the mixtures obtained as sublimates or distillates and containing 30-99.9 % by weight of TAED, about 0.1-50 % by weight of triAED and about 0.01-20 % by weight of DAED, are dispersed in the suspending agents water, aqueous solutions of neutral salts, carboxylic acids or carboxylic acid anhydrides, which are used by themselves or as mixtures of more than one of these, at − 20 to + 100 °C, the particles of the product mixture being brought to an average size of 1-2,000 μm before or during the dispersion, and after a residence time of from 0.1 minutes to several days the TAED is isolated from the suspending agent by known methods.

**Revendications**

1. Procédé de production de tétracétyl-éthylène diamine (TAED) purifiée, caractérisé en ce qu'on disperse des mélanges contenant de la TAED, qui ont été obtenus dans la fabrication de la TAED par acétylation de l'éthylène diamine ou de l'éthylène diamine acétylée, dans les agents de suspension : eau, solutions aqueuses de sels neutres, acides carboxyliques ou anhydrides d'acides carboxyliques, qui sont appliqués individuellement ou sous forme de mélange de plusieurs et qui ont une température de − 20 à + 100 °C, les particules du mélange de produits étant amenées avant ou pendant la dispersion à une dimension moyenne de 1 à 2 000 μm et, après une durée de séjour de 0,1 minute à plusieurs jours la TAED étant isolée de l'agent de suspension par des méthodes connues.

2. Procédé selon la revendication 1, caractérisé en ce qu'on utilise comme agent de suspension de l'eau, de l'acide acétique, de l'anhydride acétique, un mélange eau/acide acétique ou acide acétique/anhydride acétique.

3. Procédé selon les revendications 1 et 2, caractérisé en ce qu'on utilise de l'eau comme agent de suspension.

4. Procédé selon les revendications 1 à 3, caractérisé en ce que le rapport pondéral entre agent de suspension et mélange de produits contenant de la TAED est de 0,5 à 100 pour 1.

5. Procédé selon les revendications 1 à 4, caractérisé en ce que le mélange de produits contenant de la TAED est introduit sous la forme solide dans l'agent de suspension.

6. Procédé selon les revendications 1 à 4, caractérisé en ce qu'on introduit une masse fondue, un distillat ou un sublimat du mélange de produits contenant de la TAED dans l'agent de suspension.

7. Procédé selon les revendications 1 à 6, caractérisé en ce qu'on introduit le mélange de produits contenant de la TAED dans l'agent de suspension en une granulométrie moyenne dans l'intervalle de 1à 2 000 μm.

8. Procédé selon les revendications 1 à 7, caractérisé en ce que les substances d'accompagnement de la TAED qui subsistent dans l'agent de suspension sont recyclées à l'acétylation pour la fabrication de TAED.

9. Procédé de production de TAED purifiée, caractérisé en ce que :

1. on débarrasse les mélanges de produits contenant de la TAED, qui ont été obtenus dans la fabrication de la TAED par acétylation de l'éthylène diamine ou d'éthylène diamine acétylée, des impuretés colorantes et/ou à point d'ébullition élevé par sublimation, ou de préférence par distillation, et

2. on disperse les mélanges obtenus sous forme de sublimat ou de distillat, contenant 30-99,9 % en poids de TAED, environ 0,1 à 50 % en poids de TriAED et environ 0,01 à 20 % en poids de DAED, dans les agents de suspension : eau, solutions aqueuses de sels neutres, acides carboxyliques ou anhydrides d'acides carboxyliques, qui sont appliqués individuellement ou sous forme de mélange de plusieurs, à − 20 jusqu'environ + 100 °C, les particules du mélange de produits étant amenées avant ou pendant la dispersion à une dimension moyenne de 1 à 2 000 μm et, après un temps de séjour de 0,1 minute à plusieurs jours, la TAED étant isolée de l'agent de suspension par des méthodes connues.